(19) Europäisches Patentamt
European Patent Office
Office européen des brevets

(11) Veröffentlichungsnummer: **0 254 179 A2**

(12) # EUROPÄISCHE PATENTANMELDUNG

(21) Anmeldenummer: **87110127.5**

(22) Anmeldetag: **14.07.87**

(51) Int. Cl.[4]: **C07D 513/06**, A61K 31/435, //(C07D513/06,277:00,221:00)

Ein Antrag gemäss Regel 88 EPÜ auf Berichtigung des Beispiels 6 auf Seite 22 liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **21.07.86 DE 3624607**

(43) Veröffentlichungstag der Anmeldung: **27.01.88 Patentblatt 88/04**

(84) Benannte Vertragsstaaten: **AT BE CH DE ES FR GB GR IT LI LU NL SE**

(71) Anmelder: **BOEHRINGER INGELHEIM KG**
**D-6507 Ingelheim am Rhein(DE)**

(84) **AT BE CH DE ES FR GB GR IT LI LU NL SE**

(71) Anmelder: **Boehringer Ingelheim International G.m.b.H**
**D-6507 Ingelheim am Rhein(DE)**

(84) **GB**

(72) Erfinder: **Schneider, Claus, Dr.**
**Albrecht-Dürer-Strasse 19**
**D-6507 Ingelheim(DE)**
Erfinder: **Merz, Herbert, Dr.**
**Rotweinstrasse 53**
**D-6507 Ingelheim(DE)**
Erfinder: **Sobotta, Rainer, Dr.**
**Ludwig Richter Strasse 6**
**D-6507 Ingelheim(DE)**
Erfinder: **Bauer, Rudolf, Dr.**
**Aarstrasse 4**
**D-6200 Wiesbaden(DE)**
Erfinder: **Mierau, Joachim, Dr.**
**An den Weiden 3**
**D-6500 Mainz 33(DE)**
Erfinder: **Schingnitz, Günter, Dr.**
**Unter den Gärten 18**
**D-6550 Bad Kreuznach(DE)**

(54) **Neue Tetrahydrobenzothiazolo-chinoline ihre Herstellung und Verwendung.**

(57) Es werden neue Verbindungen der allgemeinen Formel

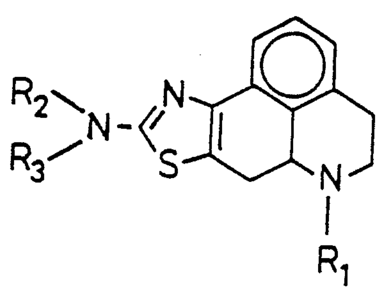

(I)

EP 0 254 179 A2

($R_1$, $R_2$ und $R_3$ sind in der Beschreibung definiert) beschrieben. Ihre Herstellung kann nach bekannten Verfahren erfolgen. Die neuen Verbindungen eigenen sich als Wirkstoffe für Arzneimittel.

## Neue Tetrahydrobenzothiazolo-chinoline ihre Herstellung und Verwendung

Die Erfindung betrifft neue Tetrahydrobenzothiazolo-chinoline der allgemeinen Formel

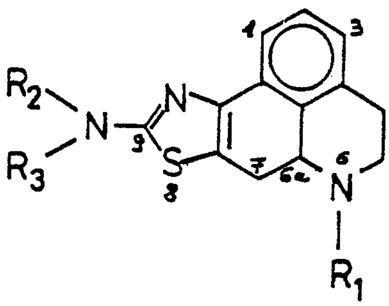

(I)

sowie ihren Enantiomeren, in Form ihrer Salze mit einer anorganischen oder organischen Säure oder als Basen, sowie Verfahren zu ihrer Herstellung und ihre Verwendung als Arzneimittel in üblichen Zubereitungen.

Es wurde gefunden, daß Verbindungen der Formel I und ihre Säureadditionssalze als Arzneimittel verwendbar sind, wenn in der allgemeinen Formel I für

$R_1$ ein Wasserstoffatom, eine geradkettige oder verzweigtkettige Alkylgruppe mit 1 bis 6 Kohlenstoffatomen, ein Allyl-oder Propargylrest, eine Phenylalkylgruppe mit 1 bis 3 Kohlenstoffatomen im Alkylteil,

$R_2$ ein Wasserstoffatom, eine geradkettige oder verzweigtkettige Alkylgruppe mit 1 bis 6 Kohlenstoffatomen, ein Allyl-oder Propargylrest, eine Phenylalkylgruppe mit 1 bis 3 Kohlenstoffatomen im Alkylteil, wobei der Phenylring durch Fluor, Chlor, Brom oder durch einen Hydroxy-, Methoxy -, Methyl-, Amino-oder durch einen Trifluormethylrest monosubstituiert oder durch diese Reste - auch in verschiedenen Kombinationen - disubstituiert sein kann, wobei der Phenylring durch einen der genannten Substituenten vorzugsweise in 4-Stellung substituiert ist, eine Alkanoylgruppe mit 1 bis 3 Kohlenstoffatomen, die auch durch einen wie oben angegebenen mono-oder disubstituierten Phenylrest substituiert sein kann,

$R_3$ ein Wasserstoffatom, eine geradkettige oder verzweigtkettige Alkylgruppe mit 1 bis 6 Kohlenstoffatomen, ein Allyl-oder Propargylrest
steht.

Die Definition von $R_2$ als Alkanoylgruppe, als gegebenenfalls substituierte Phenylalkanoylgruppe und als gegebenenfalls substituierte Phenylalkylgruppe mit 1 bis 3 Kohlenstoffatomen im Alkylteil gilt nur dann, wenn $R_3$ als H oder $CH_3$ definiert ist.

Darüberhinaus steht $R_1$ nicht für einen Methylrest, wenn beide Reste $R_2$ und $R_3$ für Wasserstoff stehen.

Bevorzugt bedeutet der Rest $R_1$

neben Wasserstoff Methyl, Ethyl, n-Propyl, iso-Propyl-,Butyle, Pentyle und Hexyle wie z.B., n-Butyl, iso-Butyl, tert-Butyl, n-Pentyl, iso-Pentyl, n-Hexyl, iso-Hexyl, 2,2-Dimethylbutyl, 2,3-Dimethylbutyl, 3-Methylpentyl, 4-Methylpentyl; die Benzyl-, eine Phenethyl-oder Phenylpropylgruppe,

während für die Reste $R_2$ und $R_3$
neben den oben bezeichneten Resten die

4-Chlorbenzyl-, 4-Fluorbenzyl-, 4-Brombenzyl-, 4-Hydroxybenzyl-, 4-Methoxybenzyl-, 4-Aminobenzyl-, 4-Trifluormethylbenzyl-sowie die entsprechend substituierten Phenylethyl-und Phenylpropylgruppen, die 3,4-Dichlorbenzyl-, 3,4-Difluorbenzyl-, 3,4-Dibrombenzyl-, 3,4-Dihydroxybenzyl-, 3,4-Dimethoxybenzyl-, 3,4-Dimethylbenzyl-, 3,4-Diaminobenzyl-sowie die entsprechend disubstituierten Phenylethyl-und Phenylpropylgruppen und die entsprechend gemischten disubstituierten Benzyl-, Phenylethyl-und Phenylpropylgruppen, die Benzoyl-, 4-Chlorbenzoyl-, 4-Methoxybenzoyl-, 4-Aminobenzoyl-, 4-Trifluormethylbenzoyl-, 4-Chlorphenylacetyl-, 4-Fluorphenylacetyl-, 4-Bromphenylacetyl-, 4-Hydroxyphenylacetyl-, 4-Methoxyphenylacetyl-, 4-Methylphenylacetyl-, 4-Aminophenylacetyl-, 3-(4-Chlorphenyl)propanoyl-, 3-(4-Fluorphenyl)propanoyl-, 3-(4-Bromphenyl)propanoyl-, 3-(4-Hydroxyphenyl)propanoyl-, 3(4-Aminophenyl)-propanoyl-,3-(4-Methoxyphenyl)propanoyl-, Formyl-, Acetyl-oder Propionylgruppe

in Betracht kommen.

Als besonders bevorzugte Verbindungen der obigen allgemeinen Formel I gelten Verbindungen, in denen

$R_1$ ein Wasserstoffatom, eine Alkylgruppe mit 1 bis 4, vor allem 3 Kohlenstoffatomen, eine Phenylalkylgruppe mit 1 bis 2 Kohlenstoffatomen im Alkylteil,

$R_2$ ein Wasserstoffatom, eine Alkylgruppe mit 1 bis 3 Kohlenstoffatomen, eine Alkanoylgruppe mit 1 bis 3 Kohlenstoffatomen, die substituiert sein kann durch einen Phenylrest oder einen durch Chlor, Brom, Fluor, eine Hydroxy-, Methoxy-, Methyl-, Amino-oder eine Trifluormethylgruppe mono-oder disubstituierten Phenylrest,

$R_3$ ein Wasserstoffatom oder eine Alkylgruppe mit 1 bis 3 Kohlenstoffatomen
bedeuten, wobei $R_1$ nicht für einen Methylrest steht, wenn sowohl $R_2$ als auch $R_3$ Wasserstoff bedeutet

Die neuen Verbindungen können als Racemate oder reine Enantiomere, aber auch als Gemische der Enantiomeren in beliebigen Verhältnissen vorliegen.

Von Berney, D. und Schuh, K. sind verschiedene Strukturanaloga des Apomorphins synthetisiert worden, von denen die Pyrrolverbindung

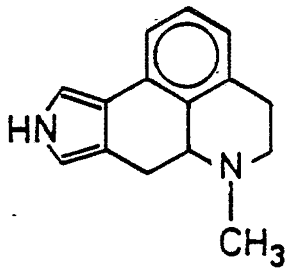

und die Pyrazolverbindung

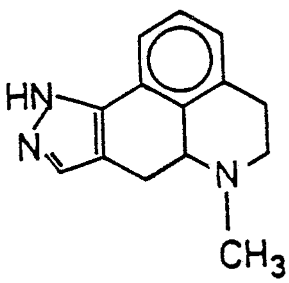

als näher benannte Verbindungen eine dem Apomorphin ähnliche dopaminerge Wirkung haben sollen (Berney, D., Schuh, K., Helvetica Chimica Acta, 65, 1304-9, 1982).

Die klinische Verwendung von Apomorphin ist jedoch wegen der geringen therapeutischen Breite nur sehr eingeschränkt möglich.

Die Verbindung der Formel

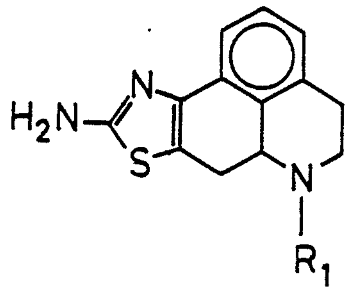

(II)

$(R_1 = CH_3)$

ist bekannt (Berney, D., Schuh, K., s.o.). Über eine etwaige dopaminerge Wirkung dieser Verbindung wird aber keine Aussage gemacht.

Die zu lösende Aufgabe bestand darin, neue dopaminerg wirkende Verbindungen vorzuschlagen, die einen gezielten, möglichst nebenwirkungsfreien therapeutischen Einsatz ermöglichen.

Überraschenderweise haben wir gefunden, daß die erfindungsgemäßen Verbindungen der Formel I eine starke, hochselektive präsynaptische dopaminagonistische Wirkung entfalten und gegenüber dem Stand der Technik gezieltere und effektivere therapeutische Einsatzmöglichkeiten eröffnen.
Die gesuchte Wirkung findet sich vorwiegend bei einer von zwei enantiomeren Formen, im allgemeinen beim (-)-Enantiomeren.
Für den Einfluß der Substituenten auf die Wirkungsqualitäten der neuen Verbindungen lassen sich gewisse Trends beobachten. So können sich Kettenverlängerungen positiv auswirken. Beispielsweise hat sich gezeigt, daß -bei unveränderter Bedeutung von $R_2$ und $R_3$ (z.B. beide Wasserstoff) und Variation von $R_1$-- schon die Kettenverlängerung von Methyl über Ethyl zu Propyl eine Wirkungssteigerung mit sich bringt; das zeigt sich u.a. in der erhöhten Dopaminutilisation (siehe unten).

Zur Herstellung der neuen Verbindungen der Formel I dienen an sich bekannte Verfahren:

1. Man halogeniert ein Benzochinolinverbindung der Formel

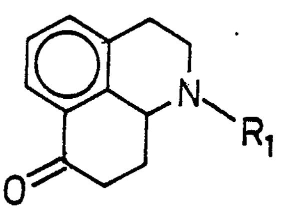

(III),

worin $R_1$ die obige Bedeutung hat, vorzugsweise mit Brom, und setzt anschließend das erhaltene Halogenderivat der Verbindung III mit einem Thioharnstoff der allgemeinen Formel

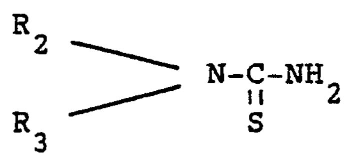

(IV),

mit den wie oben beschriebenen Resten $R_2$ und $R_3$, um.

Die Halogenierung kann innerhalb eines weiten Temperaturbereichs,zweckmäßig bei 0-100°C, vorzugsweise bei 15 - 35°C und vorteilhaft in einem geeigneten Lösungsmittel, wie z.B. Eisessig, chlorierte Kohlenwasserstoffe, niedere aliphatische Alkohole wie Methanol oder Ethanol, erfolgen.
Die Umsetzung des Halogenderivats der Verbindung III mit IV erfolgt zwischen 0° und 150°C, vorzugsweise zwischen 50 und 70°C in dem bei der Halogenierung benutzten oder einem anderen geeigneten Lösungsmittel, z.B. niedere Alkohole.

Die Ausgangsstoffe der Formel III sind bekannt oder können nach üblichen Verfahren gewonnen werden. Das folgende Syntheseschema zeigt die Herstellung der Ausgangsstoffe der Formel III und den be-

schriebenen Reaktionsweg:

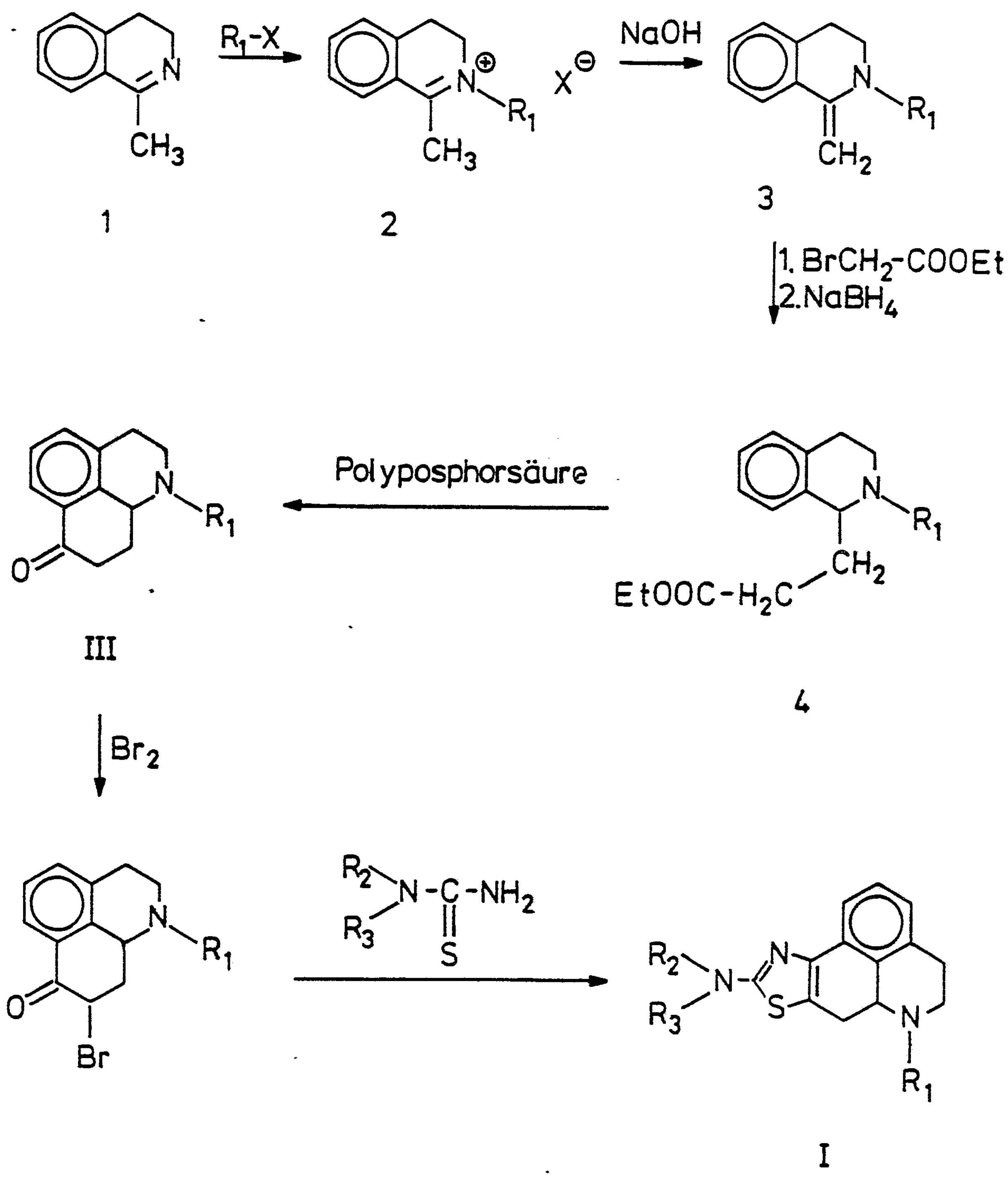


1 2 3 4 III I

2. Entalkylierung einer Verbindung der Formel

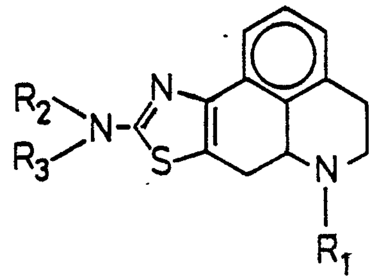 (I) ,

worin $R_1$ eine nach bekannten Entalkylierungsverfahren zu entfernende und durch Wasserstoff zu ersetzende Gruppe, vorzugsweise Methyl oder Benzyl, bedeutet und $R_2$ und $R_3$ die oben beschriebene Bedeutung haben. Die Entmethylierung läßt sich zweckmäßig z.B. oxidativ mit Perbenzoesäure und Eisen-(II)-chlorid, die Entbenzylierung am einfachsten katalytisch mit Wasserstoff in Gegenwart von Palladium-Kohle durchführen.

3. Amidierung einer Verbindung der Formel

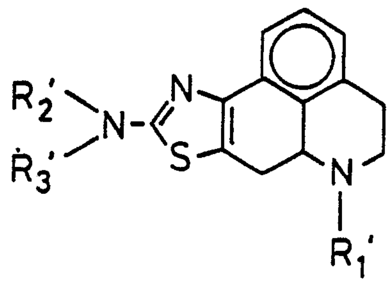 (I') ,

worin $R_2'$ und $R_3'$ Wasserstoff sind und $R_1'$ die für $R_1$ in Formel I beschriebene Bedeutung hat, mit einem Säurechlorid der Formel

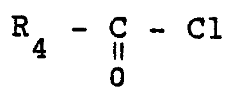

worin $R_4$ eine der oben beschriebenen Alkyl-oder Phenylgruppen in den für $R_2$ beschriebenen Acyl-oder Phenylacylresten bedeuten kann.

Die Umsetzung erfolgt in einem weiten Temperaturbereich, vorzugsweise bei 0-100°C, in einem inerten Lösungsmittel, wie Tetrahydrofuran, Dimethylformamid, Ethanol, chlorierte Kohlenwasserstoffe, in Gegenwart eines säurebindenden Mittels wie $NaHCO_3$, $Na_2CO_3$, $K_2CO_3$, CaO oder Triethylamin, Ethyldicyclohexylamin, Pyridin, unter Rückflußtemperatur.

4. Reduktion einer Verbindung der Formel

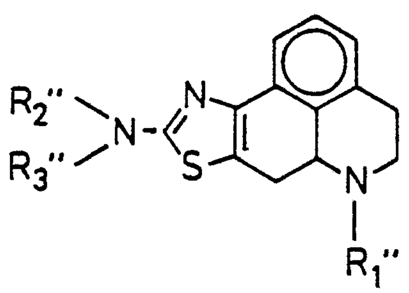 (I'') ,

worin $R_1''$ die für $R_1$ in Formel I beschriebene Bedeutung hat und $R_2''$ Acyl-oder Phenylacylreste und $R_3''$ Wasserstoff sind, in einem geeigneten Lösungsmittel, wie beispielsweise Ether, Dioxan, vorzugsweise

Tetrahydrofuran, mit einem komplexen Metallhydrid, vorzugsweise Lithiumaluminiumhydrid, bei Temperaturen zwischen -10 und 150°C, vorzugsweise bei 0 bis 20°C.

Das folgende Syntheseschema zeigt die oben beschriebenen Reaktionswege 2., 3. und 4.:

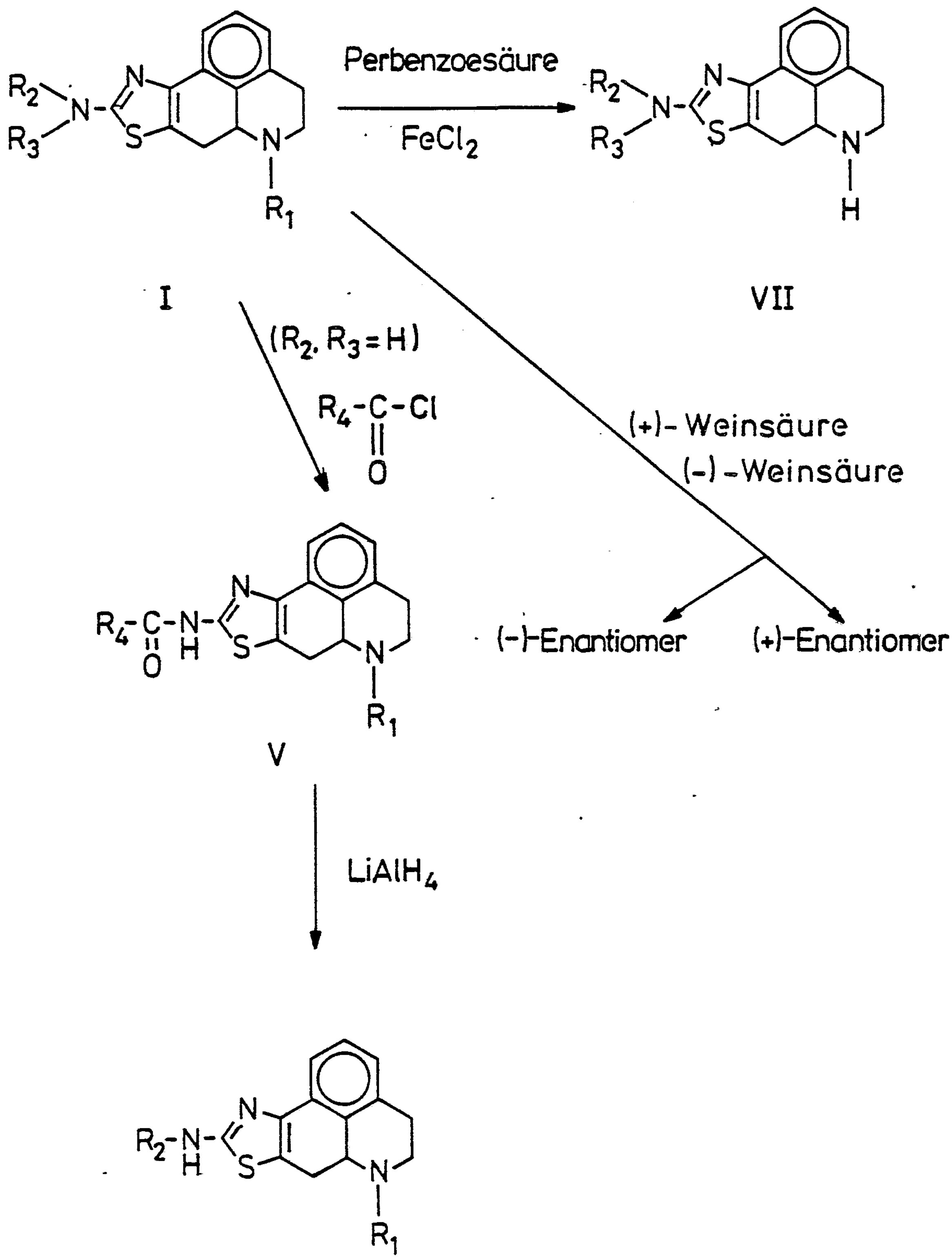


I

VII

V

VI

Die erhaltenen racemischen Verbindungen der allgemeinen Formel I lassen sich nach üblichen Methoden in ihre Enantiomeren auftrennen, beispielsweise durch Säulenchromatographie an einer chiralen Phase, oder durch Trennung ihrer diastereomeren Salze mit optisch aktiven Hilfssäuren wie Weinsäure, 0,0-Dibenzoyl-weinsäure, Camphersäure, Camphersulfonsäure oder α-Methoxy-phenylessigsäure mittels fraktionierter Kristallisation oder mittels Säulenchromatographie ihrer Konjugate.

Die erhaltenen Verbindungen der Formel I lassen sich in ihre Säureadditionssalze, insbesonder in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure, Fumarsäure oder Oxalsäure in Betracht. Soweit zunächst Salze erhalten werden, können diese nach üblichen Methoden in die freien Basen überführt werden.

Die Verbindungen der allgemeinen Formel I sind den aus dem Stand der Technik bekannten Verbindungen in ihren pharmakologischen Eigenschaften deutlich überlegen. Besonders wertvoll sind die (-)-Enantiomeren, vorzugsweise mit $R_1$ als Ethyl-oder Propylgruppe.Die neuen Verbindungen und ihre physiologisch verträglichen Säureadditionssalze eignen sich aufgrund ihrer überraschenden und wertvollen pharmakologischen Eigenschaften insbesondere für eine therapeutische Anwendung bei zentralnervösen, neuropsychiatrischen Erkrankungen, besonders der Schizophrenie, bei der Parkinsonschen Krankheit und bei einer Prolactin-Überproduktion.

Im Vergleich zu der bekannten racemischen Verbindung 6-Methyl-9-amino-5,6,6a,7-tetrahydro-4H-benzo[de]thiazolo[4,5-g] chinolin

wurden daher beispielsweise die Verbindungen ((-)-Enantiomere)

a = 9-Amino-5,6,6a,7-tetrahydro-4H-benzo[de]thiazolo[4,5-g] chinolin
b = 6-Ethyl-9-amino-5,6,6a,7-tetrahydro-4H-benzo[de]thiazolo[4,5-g]chinolin.
c = 6-Propyl-9-amino-5,6,6a,7-tetrahydro-4H-benzo[de]thiazolo[4,5-g]chinolin
d = 6-Methyl-9-benzylamino-5,6,6a,7-tetrahydro-4H-benzo[de]thiazolo[4,5-g]chinolin
e = 6-Ethyl-9-methylamino-5,6,6a,7-tetrahydro-4H-benzo[de]thiazolo[4,5-g]chinolin
f = 6-Ethyl-9-dimethylamino-5,6,6a,7-tetrahydro-4H-benzo[de]thiazolo[4,5-g]chinolin. hinsichtlich ihrer Beeinflussung präsynaptischer Dopaminrezeptoren untersucht, wobei zunächst die Wirkung auf die exploratorische Aktivität von Mäusen getestet und anschließend die Beeinflussung der Dopaminutilisation und der Dopaminsynthese an Ratten wie folgt bestimmt wurden:

1. Hemmung der exploratorischen Aktivität

Die Aktititätsmessung erfolgte in Beobachtungskäfigen, die mit einer Infrarotlichtschranke versehen sind. Gemessen wird die Häufigkeit der Unterbrechung des Lichtstrahles durch eine Gruppe von 5 Mäusen innerhalb von 5 Minuten. Gruppen von jeweils 5 Tieren erhalten die zu untersuchende Substanz in einer Dosis von 10 mg/kg subkutan injiziert. 1 Stunde später werden die Tiere in die Beobachtungskäfige gebracht, wo sofort mit der Messung der exploratorischen Aktivität über 5 Minuten begonnen wird. Parallel bzw. alternierend zu Gruppen, die mit Testsubstanz behandelt wurden, werden kochsalzbehandelte Kontrollgruppen (o,9%ig; 0,1 ml/10 g Körpergewicht subkutan) untersucht.

Alle diejenigen Substanzen wurden in die weitere Versuchsreihe aufgenommen, die als Vorbedingung eine Hemmung der exploratorischen Aktivität gegenüber den Kontrolltieren von >50% aufwiesen, wie es beispielsweise für die oben näher bezeichneten Verbindungen a,b,c,d,e, und f nachzuweisen war.

2. Bestimmung der Dopaminutilisation

Die Dopaminutilisation wurde an Ratten gemessen. Bei Tieren, die mit α-Methylparatyrosin (AMPT) (250 mg/kg intraperitoneal) behandelt werden, fällt die Dopaminkonzentration im Gesamthirn mit fortschreitender Versuchsdauer ab. Durch Substanzen, die an Autorezeptoren wirken, kann der Dopaminabfall (im Vergleich zu mit Kochsalzlösung behandelten Kontrolltieren) vermindert werden.

Die Testsubstanzen werden zum Zeitpunkt 0 und 2 Stunden des Experiments - soweit nicht anders angegeben - zur Erstellung einer Dosis-Wirkungskurve mit 0,01 bis 10 mg/kg s.c. appliziert. Zum Zeitpunkt 4 Stunden des Experiments werden die Tiere getötet und die Gehirne der Dopaminbestimmung mittels Hochdruckflüssigkeitchromatographie mit elektrochemischer Detektion zugeführt. Bestimmt wird die durch die Testsubstanz bewirkte prozentuelle Steigerung der Dopaminutilisation nach AMPT-Behandlung.

Zum Beispiel wurden folgende Ergebnisse erhalten :

| Substanz | Dosis [mg/kg] s.c. | Prozentuale Steigerung der Dopaminutilisation |
|---|---|---|
| Vergleich | 10 | 34 |
| b | 10 | 64 |
| c | 10 | 112 |

Nebenwirkungen traten in den bisher angewandten Dosierungen nicht in Erscheinung.

Ferner sind die erfindungsgemäß hergestellten Verbindungen weitgehend untoxisch. So konnten bei der Untersuchung der Substanzen an Ratten mit Dosen zwischen 100 und 200 mg/kg s.c. keine Todesfälle festgestellt werden.

Aufgrund ihrer pharmakologischen Eigenschaften eignen sich die erfindungsgemäß hergestellten Verbindungen der allgemeinen Formel I und ihre physiologisch verträgliche Säureadditionssalze bei der Behandlung von zentralnervösen, neuropsychiatrischen Erkrankungen, insbesondere der Schizophrenie, bei der Behandlung der Parkinsonschen-Krankheit und bei einer Prolactin-Überproduktion.

Zur therapeutischen Anwendung lassen sich die neuen Verbindungen und ihre physiologisch verträglichen Säureadditionssalze, gegebenfalls in Kombination mit anderen Wirkstoffen, in die üblichen galenischen Anwendungsformen wie Dragées, Tabletten, Pulver, Suppositorien, Suspensionen, Tropfen oder Ampullen einarbeiten. Die Einzeldosis beträgt hierbei 1 bis 4 $^{\times}$ täglich 0,01 - 5 mg/kg Körpergewicht, vorzugsweise jedoch 0,1 - 3 mg/kg Körpergewicht.

Als Beispiele für Arzneimittelzubereitungen werden genannt:

Beispiel I: Dragées

| | |
|---|---|
| 5,0 Teile | Wirkstoff gemäß der Erfindung |
| 33,5 Teile | Milchzucker |
| 10,0 Teile | Maisstärke |
| 1,0 Teile | Gelatine |
| 0,5 Teile | Magnesiumstearat |

Die pulverisierten Bestandteile Wirkstoff, Milchzucker und Maisstärke werden mit wäßriger Gelatinelösung granuliert und getrocknet. Das Granulat wird mit dem Magnesiumstearat vermischt und zu Dragéekernen von 50 mg Gewicht verpreßt und nach bekannten Methoden überzogen.

Beispiel II: Suppositorien

| | |
|---|---|
| 10 Teile | Wirkstoff gemäß der Erfindung |
| 1690 Teile | Zäpfchenmasse (z.B: Witepsol W 45) |

Die feingepulverte Substanz wird mittels eines Homogenisators in der geschmolzenen, auf 40°C abgekühlten Zäpfchenmasse gleichmäßig verteilt. Aus der Mischung werden Zäpfchen mit einem Gewicht von 1,7 g geformt.

Beispiel III:Tropfen

| | |
|---|---|
| 0,035 Teile | p-Hydroxybenzoesäure-methylester |
| 0,015 Teile | p-Hydroxybenzoesäure-n-propylester |
| 0,05 Teile | Anisöl |

| | | |
|---|---|---|
| | 0,06 Teile | Menthol |
| | 10,0 Teile | Ethanol rein |
| | 0,5 Teile | Wirksubstanz gemäß der Erfindung |
| | 0,7 Teile | Zitronensäure |
| | 0,3 Teile | Natriumphosphat sek. $\times$ 2 $H_2O$ |
| | 1,0 Teile | Natriumcyclamat |
| | 15,0 Teile | Glycerin |
| ad | 100,0 ml | Dest. Wasser |

Die p-Hydroxybenzoesäureester, Anisöl sowie Menthol werden in Ethanol gelöst (Lösung I). Die Puffersubstanzen, die Wirksubstanz und Natriumcyclamat werden in dest. Wasser gelöst und Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen

Die nachfolgenden Beispiele sollen die Herstellung bekannter Zwischen-oder Ausgangsverbindungen näher erläutern

Beispiel A

1-Methyl-2-ethyl-dihydroisochinoliniumbromid (Formel 2 mit $R_1$ = $C_2H_5$).

0.1 Mol der Isochinolinylverbindung (1) als bekanntes Ausgangsprodukt wird bei Raumtemperatur mit Ethylbromid (0,1 mol) in Acetonitril (150 ml) alkyliert. Nach dem Einengen wird der feste Rückstand aus Alkohol umkristallisiert.

Ausbeute: 15,5g (61% der Theorie); Schmp. 118-121°C.

In analoger Weise zu Beispiel A wurden
1-Methyl-2-propyl-dihydroisochinolinium Bromid (Formel 2 mit $R_1 = C_3H_7$),
Ausbeute: 90% der Theorie; Schmp. 144-146°C
und 1,2-Dimethyl-dihydro-isochinolinium Iodid (Formel 2 mit $R_1 = CH_3$),
Ausbeute: 63% der Theorie; Schmp. 193-194°C
hergestellt.

Beispiel B

2-Ethyl-tetrahydro-1-isochinolinyl-propionsäureethylester (Formel 4, $R_1 = C_2H_5$)

Zu der Verbindung der Formel 2 ($R_1$ = $C_2H_5$) (31 g, 0,12 Mol), gelöst in 30 ml Wasser, werden 60 ml NaOH (2N) gegeben und bei Raumtemperatur 1 Std. gerührt. Die Reaktionsmischung wird mit Ether extrahiert, die Etherextrakte werden eingeengt. Der Rückstand wird in 300 ml Toluol gelöst, mit Bromessigester (0,12 mol) versetzt und 8 Std. am Rückfluß zum Sieden erhitzt.

Anschließend wird mit 100 ml $CH_3OH$ verdünnt. $NaBH_4$ (5,7 g) wird in 50 ml NaOH (1 N) gelöst und bei 10° C zugetropft. Die Reaktionsmischung wird 2 Std. bei Raumtemperatur gerührt, auf Wasser gegossen und mit Essigester extrahiert. Die organische Phase wird eingeengt, der Rückstand wird über Kieselgel mit Cyclohexan/Essigester (3:1) gereinigt.

Ausbeute: 26,4 g (84 % der Theorie)

Analog zu Beispiel Be wurden
2-Propyl-tetrahydro-1-isochinolinyl-propionsäureethylester (Formel 4, $R_1$ = $C_3H_7$) Ausbeute: 68% der Theorie
und
2-Methyl-tetrahydro-1-isochinolinyl-propionsäureethylester (Formel 4, $R_1$ = $CH_3$), Ausbeute 66% der Theorie,
hergestellt.

Beispiel C

1-Ethyl-hexahydro-1H-benzo[de]-chinolin-7-on (Formel III, $R_1 = C_2H_5$).

Produkt (4) mit $R_1 = C_2H_5$ (0,1 Mol) wird in 300 g Polyphosphorsäure 15 min auf 140°C erhitzt, auf Raumtemperatur abgekühlt und mit Eis versetzt. Die saure Lösung wird mit Ammoniak alkalisch gemacht und mit Essigester extrahiert. Nach dem Einengen des Lösungsmittels wird der Rückstand über Kieselgel mit Methylenchlorid/Essigester/Methanol (70 : 20 : 10) gereinigt. Ausbeute: 15,4 g (75% der Theorie);

Analog zu Beispiel C wurden
1-Propyl-hexahydro-1H-benzo[de]chinolin-7-on (Formel III, $R_1 = C_3H_7$) Ausbeute , 82% der Theorie und
1-Methyl-hexahydro-1H-benzo[de]chinolin-7-on (Formel III, $R_1 = CH_3$), Ausbeute 82% der Theorie,
hergestellt.

Die folgenden Beispiele sollen die Herstellung der erfindungsgemäßen Verbindungen näher erläutern :

Beispiel 1:

6-Ethyl-9-amino-5,6,6a,7-tetrahydro-4H-benzo[de]thiazolo[4,5-g] chinolin

0,02 Mol der Verbindung III mit $R_1$ als $C_2H_5$ werden in Eisessig gelöst und bei Raumtemperatur mit 3.2g Brom bromiert. Nach dem Einengen wird der Rückstand in 50 ml Eisessig gelöst und mit 1.5 g Thioharnstoff der allgemeinen Formel IV mit $R_2$ und $R_3$ = H 1 Std. unter Rückfluß erhitzt. Anschließend wird auf Eiswasser gegossen, mit Ammoniak alkalisch gemacht und mit Essigester extrahiert. Nach dem Einengen wird der Rückstand über Kieselgel mit Methylenchlorid/Essigester/Methanol (70 : 20 : 10) gereinigt und aus Essigester umkristallisiert.
Ausbeute. 1.6 g (30 % der Theorie); Schmp. 158 - 160° C

Beispiel 2:

6-Propyl-9-amino-5.6,6a,7-tetrahydro-4H-benzo[de]thiazolo[4,5-g] chinolin

In Analogie zu Beispiel 1 wurde diese Verbindung mit einer Ausbeute von 21% der Theorie, Schmp. 184-186°C, hergestellt.

Beispiel 3:

6-Ethyl-9-methylamino-5,6,6a,7-tetrahydro-4H-benzo[de]thiazolo [4,5-g] chinolin

In Analogie zu Beispiel 1 wurde diese Verbindung als Dihydrochlorid Halbhydrat (aus Monomethylthioharnstoff mit einer Ausbeute von 36% der Theorie, Schmp. 255-257°C, hergestellt.

Beispiel 4:

6-Ethyl-9-dimethylamino-5,6,6a,7-tetrahydro-4H-benzo[de]thiazolo[4,5-g] chinolin

In Analogie zu Beispiel 1 wurde diese Verbindung als Dihydrochlorid (aus Dimethylthioharnstoff) mit einer Ausbeute von 44% der Theorie, Schmp.> 260°C, hergestellt.

Beispiel 5:

6-Methyl-9-benzoylamino-5,6,6a,7-tetrahydro-4H-benzo[de]thiazolo[4,5-g]chinolin

0,01 Mol der Verbindung II wird in 100 ml Tetrahydrofuran gelöst und mit 1.5 g Triethylamin und 1,5 g Benzoylchlorid 3 Std. unter Rückfluß erhitzt. Anschließend wird auf Wasser gegossen und mit Methylenchlorid extrahiert. Nach dem Einengen kristallisiert der Rückstand in Ether.
Ausbeute: 3g (83 % der Theorie); Schmp. 237 - 240°C

Beispiel 6

6-Methyl-9-benzylamino-5,6,6a,7-tetrahydro-4H-benzo[de]thiazolo[4,5-g] chinolin

0,005 Mol der Verbindung II wird in 50 ml Tetrahydrofuran mit 0,5 g Lithiumaluminiumhydrid versetzt und 7 Std am Rückfluß erhitzt. Nach dem Abkühlen wird 20 ml einer gesättigten Diammoniumtartratlösung zugeben. Mit Essigester wird ausgeschüttelt und eingeengt. Der Rückstand wird über Kieselgel mit Methylenchlorid/Essigester/Methanol (70 : 20 : 10) gereinigt. Das Produkt kristallisiert in Ether.
Ausbeute: 1,2, g (63 % der Theorie), Schmp. 95-96°C.

Beispiel 7

9-Amino-5,6,6a,7-tetrahydro-4H-benzo[de]thiazolo[4,5-g] chinolin

Diese Verbindung als Dihydrochlorid erhält man durch Entmethylierung der Verbindung II ($R_1 = CH_3$) mit Perbenzoesäure und Eisen-II-chlorid. Die Durchführung erfolgt nach der Methode von Monkovic (Monkovic, I., et al., Synthesis, 770, 1985).
Ausbeute : 18% der Theorie, Schmp.>260° C

Beispiel 8

Die unter Beispiel 7 genannte erfindungsgemäße Verbindung läßt sich durch Entmethylierung von Verbindung II auch dadurch herstellen, indem 2.6 g (0,01 Mol) der Verbindung II, 2 g (0,01 Mol) m-Chlorperbenzoesäure, 150 ml Methylenchlorid, 0.7 ml Wasser und $FeCl_2$ wie folgt umgesetzt werden: Verbindung II wird, in Methylenchlorid gelöst, vorgelegt. Chlorperbenzoesäure wird bei -15 bis -10°C portionweise zugegeben. Die vollständige Umsetzung erfolgt innerhalb von 10 min, was dünnschichtchromatographisch (DC) (Methylenchlorid, Essigester, Methanol, 70:20:10; Methylenchlorid/Methanol, 80:20) nachweisbar ist. Die $FeCl_2$-Lösung (50 mg in 0,7 ml $H_2O$) und nach einer halben Stunde ca. 50 mg festes $FeCl_2$ werden dazugegeben. Die anfänglich weißen Kristalle des N-Oxids des Endprodukts färben sich langsam grün (14 h RT). Laut DC erfolgt 50%ige Umsetzung. Ca 30 ml 2N NaOH und 50 ml $CH_2Cl_2$ werden dazugegeben, über Kieselgur abgesaugt, mit $CH_2Cl_2$ durchgeschüttelt, getrocknet, eingeengt und über Kieselgur chromatographiert.
Ausbeute: ca. 150 mg, Schmp.>260°C

Beispiel 9

Die unter Beispiel 7 genannte Verbindung sowie deren Enantiomeren können auch nach folgenden Reaktionsschema erhalten werden:

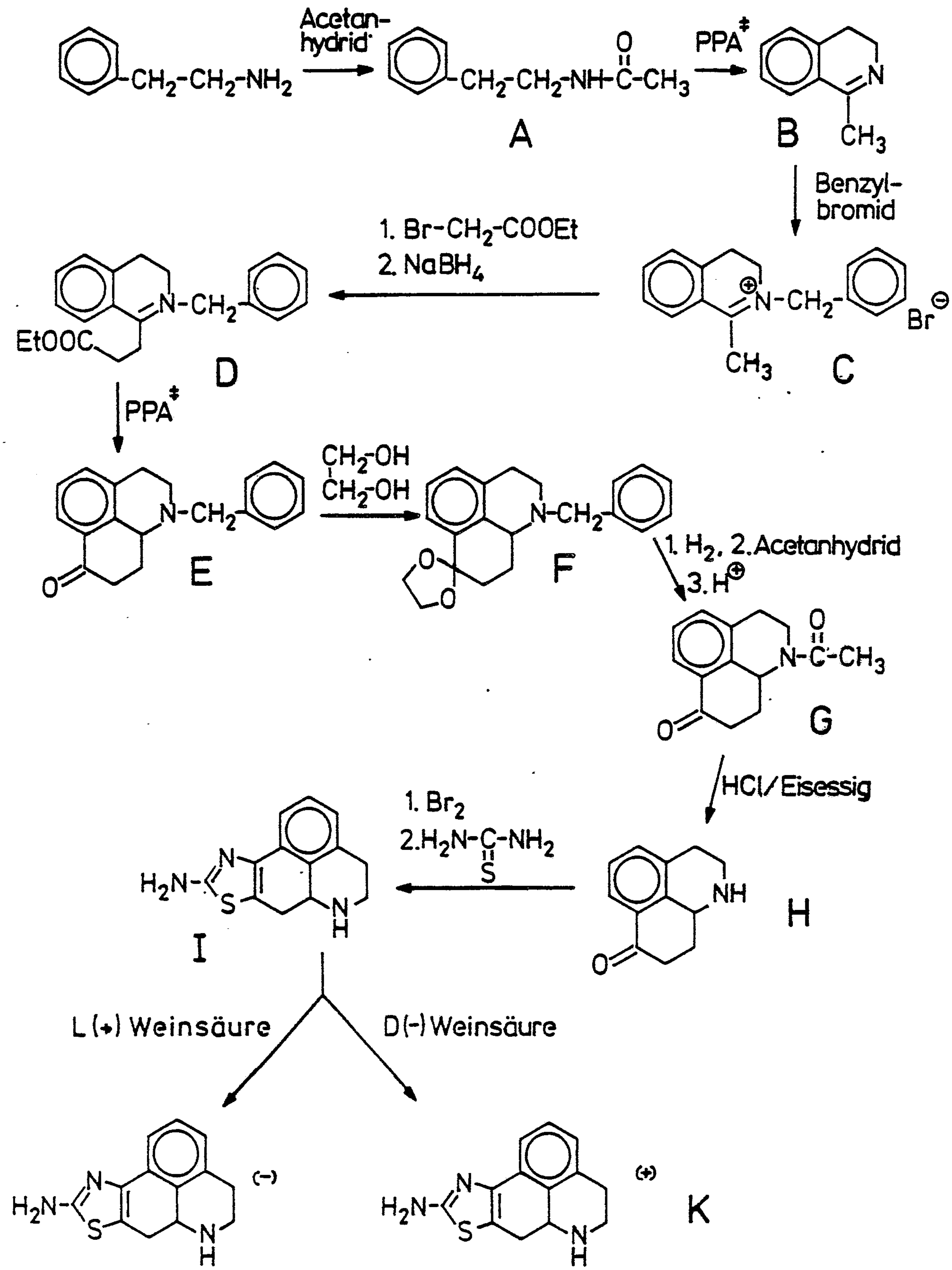


‡ PPA = Polyphosphorsäure

Die in diesem Reaktionsschema bezeichneten Verbindungen A bis K werden wie folgt hergestellt:

Verbindung A

242g Phenethylamin (2 Mol) wurden in Tetrahydrofuran vorgelegt, 204 g Acetanhydrid bei 15-20°C zugetropft und nach 1/2h bei Raumtemperatur (RT) eingeengt. Der Rückstand wurde in Essigester gelöst, 1 mal mit Ammoniak, 1 mal mit 2n HCl und 1 mal mit Wasser gewaschen. Die organische Phase wurde getrocknet und eingeengt. Ausbeute: 312 g (95.7% der Theorie)

Verbindung B

312 g der Verbindung A (1.91 Mol) und 1 kg Polyphosphorsäure (PPA) wurden 1/2h bei 200-220°C gerührt, auf 100°C abgekühlen gelassen, mit Eis versetzt, mit Ammoniak neutralisiert und mit Essigester ausgeschüttelt. Die organische Phase wurde getrocknet, eingeengt und im Hochvakuum destilliert. Ausbeute: 166 g (60% der Theorie), $K_{PO.1mm}$ : 60-62°C

Verbindung C

166 g der Verbindung B (1.14 Mol) wurden mit 390 g Benzylbromid (2.28 Mol) und 1.7 l Nitromethan 2 Stunden unter Rückfluß gerührt, eingeengt und der Rückstand in Aceton abgesaugt.
Ausbeute: 254 g (70% der Theorie), Schmp.: 203-205°C

Verbindung D

254 g der Verbindung C (0,8 Mol) wurden in 420 ml Wasser gelöst und 420 ml 2 n NaOH dazugegeben. Es wurde mit Ether ausgeschüttelt, die Etherphase getrocknet und eingeengt. Der Rückstand wurde in 800 ml Dimethylformamid (DMF) gelöst, 134 g Bromessigester (0.8 Mol) dazugegeben, für 6 Stunden bei RT gerührt und mit 500 ml Methanol verdünnt. 30 g $NaBH_4$ (0,8 Mol) wurde portionsweise dazugegeben, bei RT 1 Stunde gerührt, auf Eiswasser gegossen und mit Essigester ausgeschüttelt. Die organische Phase wurde getrocknet, eingeengt und über ca. 2 kg Kieselgel chromatographiert (Essigester/Cyclohexan, 1:3).
Ausbeute: 231 g (89% der Theorie).

Verbindung E

231 g der Verbindung D (0,71 Mol) wurden mit 2.3 kg PPA 1/2 Stunde bei 140-150°C gerührt, mit Eis versetzt, mit $NH_4OH$ alkalisch eingestellt und mit Essigester ausgeschüttelt. Die organische Phase wurde getrocknet, eingeengt und in Cyclohexan/Essigester (3:1) chromatographiert.
Ausbeute: 118.5 g (60% der Theorie)

Verbindung F

118,5 g der Verbindung E (0,43 Mol), 89.8 g p-Toluol-sulfonsäure (0,47 Mol), 29,6 g Ethylenglycol (0,47 Mol) und 3 l Toluol wurden 24 h am Wasserabscheider gekocht, auf Eiswasser gegossen, mit Ammoniak alkalisch eingestellt und mit Essigester ausgeschüttelt. Die organische Phase wurde getrocknet und eingeengt.
Ausbeute : 96.5g (70% der Theorie).

Verbindung G

96.5 g der Verbindung F (0.3 Mol) wurden mit 4.5 g Pd/C (10%), 965 ml Tetrahydrofuran und 65 g Acetanhydrid 3 Stunden bei 60-70°C und 5 bar hydriert. Der Katalysator wurde abgesaugt, die Lösung eingeengt und der Rückstand in Eisessig/2n HCl (je 1.8 Liter) gelöst. Bei RT wurde 1/2 Stunde gerührt, auf Eis gegossen, mit Ammoniak alkalisch eingestellt und mit Essigester extrahiert. Die organische Phase wurde getrocknet, eingeengt und der Rückstand in Ether abgesaugt.
Ausbeute: 55 g (80% der Theorie)

Verbindung H

55g der Verbindung G (0,24 Mol), 550 ml Eisessig und 550 ml konzentrierte HCl wurden 24 Stunden unter Rückfluß gerührt (ca. 100°C), auf Eis gegossen, mit Ammoniak alkalisch eingestellt und mit Essigester ausgeschüttelt. Die organische Phase wurde getrocknet, eingeengt, der Rückstand in Aceton gelöst, etherische HCl zugegeben und das Hydrochlorid abgesaugt.
Ausbeute: 32,3 g (60% der Theorie), Fp: >260°C

Verbindung I

32,3g der Verbindung H (0,14 Mol) wurde in 1,6 l Eisessig suspendiert vorgelegt und 23 g Brom (0,14 Mol) zugetropft. es wurde auf 30°C erwärmt, 10 min bei 30°C nachgerührt und 44 g Thioharnstoff (0,58 Mol) dazugegeben. Danach wurde 1 Stunde unter Rückfluß gerührt, auf Eis gegossen, mit konzentiertem Ammoniumhydroxid ($NH_4OH$) alkalisch eingestellt und mit Essigester ausgeschüttelt. Die organische Phase wurde getrocknet, eingengt und der Rückstand in Methanol abgesaugt.
Ausbeute: 12,5 g (35.7% der Theorie)
Fp: 159°C (Zersetzung)

Verbindungen K (Enantiomere von Verbindung I)

a. (-) Enantiomer

12.5 g der Verbindung I (0.051 Mol), 7.7 g L(+)Weinsäure (0.051 Mol) wurden in 2 Liter Wasser heiß gelöst, mit Aktivkohle versetzt und über Kieselgur abgesaugt. Über Nacht wurde auskristallisieren gelassen, abgesaugt und mit Wasser gewaschen. Man erhielt 10.3 g des gebildeten Tartrats. Diese 10.3 g wurden 4 × aus Wasser umkristallisiert und erhielt 2,5 g des reinen Tartrats. Die Base, in Wasser gelöst, mit Ammoniak alkalisch gemacht und mit Essigester ausgeschüttelt, wurde daraus freigesetzt und in Ethanol das entsprechende Hydrochlorid gebildet.
Ausbeute: 1.3 g,
$[\alpha]_D^{25}$ = (-)110°,c = 0.1, Wasser
($[\alpha]_D^{25}$ = (-)90°, c = 0.1, Aceton)
Base
Fp : 330°C (Zersetzung)

b. (+) Enantiomer

Aus den wässrigen Mutterlaugen (alle) wurden die Basen analog freigesetzt, man erhielt 8.7 g. Diese 8.7 g (0.036 Mol) wurden mit 5.4 g D(-)Weinsäure (0.036 Mol) in 1 Liter Wasser heiß gelöst, mit Aktivkohle versetzt, über Kieselgur abgesaugt und über Nacht auskristallisieren gelassen. Es wurde abgesaugt, mit Wasser gewaschen und 1 mal aus Wasser umkristallisiert. Man erhielt 3.3 g des gebildeten Tartrats.
Die Base wurde daraus wie oben beschrieben freigesetzt und das entsprechende Hydrochlorid in Ethanol gebildet.

Ausbeute: 1.9 g
$[\alpha]_D^{25}$ = (+)110°, c = 0.1, Wasser

([α] $^{25}_{D}$ = (+) 91°, c = 0.1, Aceton)
Base

Fp : 330°C (Zersetzung)
Als weitere erfindungsgemäße Verbindungen können beispielsweise hergestellt werden:

| Bsp. | $R_1$ | $R_2$ | $R_3$ | Salzform. | Schmp. °C |
|---|---|---|---|---|---|
| 10 | H- | $C_6H_5CO-$ | H- | Base | > 260 |
| 11 | $C_2H_5-$ | H- | H- | Base | 158-160°C |
| 12 | $CH_3-$ | H- | H- | 2 HCl | > 260°C (Base: 201-203°C) |
| 13 | (+)$CH_3-$ | H- | H- | 2 HCl | 274-276°C |
| 14 | (-)$CH_3-$ | H- | H- | 2 HCl | 276-277°C |
| 15 | $CH_3-$ | $C_6H_5CH_2-$ | H- | Base | 95-96°C |
| 16 | $C_2H_5-$ | $CH_3-$ | H- | 2 HCl | 255-257°C |
| 17 | $C_2H_5-$ | $CH_3-$ | $CH_3-$ | 2 HCl | > 260°C |
| 18 | $CH_3-$ | $C_6H_5CO-$ | H- | Base | 237-240°C |
| 19 | n-$C_3H_7-$ | 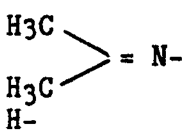 | H- | 2 HCl | 256-258°C |
| 20 | H- | H- | H- | 2 HCl | >260°C |
| 21 | (+)H- | H- | H- | 2 HCl | 330°C(Zersetzung) (Base: 223-225°C) |
| 22 | (-)H- | H- | H- | 2 HCl | 330°C(Zersetzung) (Base: 223-225°C) |
| 23 | (+)n-$C_3H_7-$ | H- | H- | 2 HCl | 274-275°C |
| 24 | (-)n-$C_3H_7-$ | H- | H- | 2 HCL | 273-275°C |

| Bsp. | $R_1$ | $R_2$ | $R_3$ | Salzform. | Schmp. °C |
|---|---|---|---|---|---|
| 25 | $C_2H_5$- | $ClC_6H_4CO$- | H- | Base | 207-210 |
| 26 | $C_2H_5$- | $ClC_6H_4CH_2$- | H- | 2 HCl | 190-192 |
| 27 | $C_2H_5$- | $C_6H_5CO$- | H- | Base | 222-224 |
| 28 | $C_2H_5$- | $CH_3OC_6H_4(CH_2)_2CO$- | H- | 2 HCl | 198-200 |
| 29 | $C_2H_5$- | $C_6H_5CH_2$- | H- | 2 HCl | 220-222 |
| 30 | $C_2H_5$- | $CH_3CO$- | H- | Base | >260°C |
| 31 | n-$C_3H_7$- | H- | H- | 2 HCl | 264-266 |
| 32 | n-$C_3H_7$- | $ClC_6H_4CO$- | H- | Base | 192-196 |
| 33 | n-$C_3H_7$- | $ClC_6H_4CH_2$- | H- | HCl | 194-197 |
| 34 | n-$C_3H_7$- | $CH_3CO$- | H- | 2 HCl | 258-260 |
| 35 | n-$C_3H_7$- | $CH_3OC_6H_4(CH_2)_2CO$- | H- | 2 HCl | 211-213 |
| 36 | n-$C_3H_7$- | $C_6H_5CO$- | H- | Oxalat | 258-259 |
| 37 | n-$C_3H_7$- | $C_6H_5CH_2$- | H- | 2 HCl | 240-242 |
| 38 | n-$C_3H_7$- | $C_2H_5$- | H- | 2 HCl | 254-255 |
| 39 | n-$C_3H_7$- | 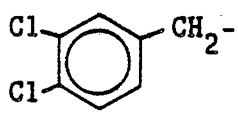 | H- | | |
| 40 | n-$C_3H_7$ | 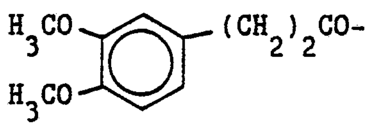 | H- | | |
| 41 | i-$C_3H_7$- | H- | H- | | |
| 42 | n-$C_4H_9$- | H- | H- | | |
| 43 | $CH_2$=CH-$CH_2$- | $C_2H_5$- | $C_2H_5$- | | |
| 44 | CH≡C-$CH_2$- | H- | H- | | |
| 45 | $C_2H_5$- | $HOC_6H_4(CH_2)_2CO$- | H- | | |
| 46 | n-$C_6H_{13}$- | H- | H- | | |

| Bsp. | $R_1$ | $R_2$ | $R_3$ | Salzform | Schmp.°C |
|---|---|---|---|---|---|
| 47 | i-$C_3H_7$- | CHO- | H- | | |
| 48 | $C_2H_5$ | $CH_3$ | $CH_2$=CH-$CH_2$- | | |
| 49 | $C_2H_5$ | $CH_3$- | CH≡C-$CH_2$- | | |
| 50 | $C_6H_5(CH_2)_3$- | | H- | | |
| 51 | H- | n-$C_6H_{13}$- | H- | | |
| 52 | $C_2H_5$- | $CH_2$=CH-$CH_2$- | $C_2H_5$- | | |
| 53 | H- | CH≡C-$CH_2$- | H- | | |
| 54 | $CH_3$ | $C_6H_5(CH_2)_3$- | H- | | |
| 55 | $C_2H_5$ | H- | n-$C_6H_{13}$ | | |
| 56 | $C_2H_5$ | n-$C_4H_9$ | H- | | |

**Ansprüche**

1. Verbindungen der allgemeinen Formel

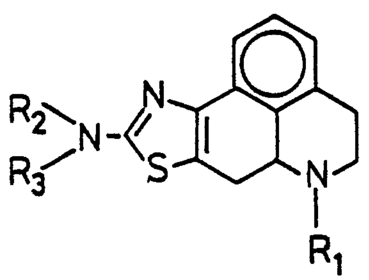

(I),

in der

$R_1$ ein Wasserstoffatom, eine geradkettige oder verzweigtkettige Alkylgruppe mit 1 bis 6 Kohlenstoffatomen, ein Allyl-oder Propargylrest, eine Phenylalkylgruppe mit 1 bis 3 Kohlenstoffatomen im Alkylteil,

$R_2$ ein Wasserstoffatom, eine geradkettige oder verzweigtkettige Alkylgruppe mit 1 bis 6 Kohlenstoffatomen, ein Allyl-oder Propargylrest, eine Phenylalkylgruppe mit 1 bis 3 Kohlenstoffatomen im Alkylteil, wobei der Phenylring durch Fluor, Chlor, Brom, Hydroxy-, Methoxy-, Methyl-,Amino-oder Trifluormethylreste monosubstituiert oder durch die genannten Reste auch in verschiedenen Kombination untereinander disubstituiert sein kann, eine Alkanoylgruppe mit 1 bis 3 Kohlenstoffatomen, die durch einen wie oben angegebenen mono - oder disubstituierten Phenylrest substituiert sein kann,

$R_3$ ein Wasserstoffatom, eine geradkettige oder verzweigtkettige Alkylgruppe mit 1 bis 6 Kohlenstoffatomen, ein Allyl - oder Propargylrest sein kann, in der für $R_1$ dann nicht die Methylgruppe steht, wenn $R_2$ und $R_3$ gleichzeitig Wasserstoff bedeuten,

in Form der Racemate, der Enantiomeren und der jeweiligen Säureaditionssalze.

2. Verbindungen der allgemeinen Formel I, dadurch gekennzeichnet, daß

$R_1$ ein Wasserstoffatom, eine geradkettige oder ungeradkettige Alkylgruppe mit 1 bis 4 Kohlenstoffatomen, Benzyl - oder Phenylethylgruppe

$R_2$ ein Wasserstoffatom, eine geradkettige oder ungeradkettige Alkylgruppe mit 1 bis 3 Kohlenstoffatomen, Benzyl-, 4-Chlorbenzyl-, 3,4-Dichlorbenzyl-, Phenyl-ethyl-, eine Alkanoylgruppe mit 1 bis 3 Kohlenstoffatomen-, die durch einen Phenyl-oder einen mit Fluor, Chlor, Brom, Hydroxy-, Methoxy-oder Trifluormethylrest substituierten Phenylrest substituiert sein kann, wobei $R_2$ nur dann Alkanoyl-oder substituierte Phenylalkanoyl-oder Phenylalkyl-oder substituierte Phenylalkylgruppe bedeutet, wenn $R_3$ ein Wasserstoffatom oder eine Methylgruppe ist,

$R_3$ ein Wasserstoffatom, eine Methyl-, Ethyl-, n-Propyl-oder i-Propylgruppe bedeutet, ihre Enantiomeren und die jeweiligen Säureadditionssalze

3. Verbindungen der allgemeinen Formel I, dadurch gekennzeichnet, daß

$R_1$ ein Wasserstoffatom, eine Methyl-, Ethyl-, n-Propyl-, i-Propyl -, Butyl-, Benzyl - oder Phenylethylgruppe,

$R_2$ ein Wasserstoffatom, eine Methyl-, Ethyl-, eine Alkanoylgruppe mit 1 bis 3 Kohlenstoffatomen, die durch einen Phenylrest oder einen mit Fluor, Chlor, Brom, Hydroxy-, Methoxy-oder Trifluormethylrest substituierten Phenylrest substituiert sein kann,

$R_3$ ein Wasserstoff, eine Methyl-oder Ethylgruppe bedeutet,ihre Enantiomeren und die jeweiligen Säureadditionssalze

4. Verbindungen der allgemeinen Formel I, dadurch gekennzeichnet, daß

$R_1$ ein Wasserstoffatom, eine Ethyl-, n-Propyl - oder i - Propylgruppe,

$R_2$ ein Wasserstoffatom, eine Methyl-, Ethyl-, Formyl-, Acetyl-, Propionyl-, Benzoyl-, Benzoylacetyl-, 4-Chlorbenzoyl-, 4-Chlorphenylacetyl-oder 3-(4-Methoxyphenyl)propanoylgruppe,

$R_3$ ein Wasserstoffatom, eine Methyl-oder Ethylgruppe bedeutet, ihre Enantiomeren und die jeweiligen Säureadditionssalze.

5. Verbindungen der allgemeinen Formel I gemäß der Ansprüche 1 bis 4, dadurch gekennzeichnet, daß der Phenylring der Phenylalkylgruppe oder der Phenylalkanoylgruppe von $R_2$ mit Fluor, Brom, Hydroxy-, Methoxy-, Methyl-oder einem Trifluormethylrest in 4-Stellung substituiert ist.

6. 9-Amino-5,6,6a,7-tetrahydro-4H-benzo[de]thiazolo[4,5-g] chinolin, seine Enantiomeren und die Säureadditionssalze

7. 6-Ethyl-9-amino-5,6,6a,7-tetrahydro-4H-benzo[de]thiazolo [4,5-g]chinolin, seine Enantiomeren und die Säureadditionssalze

8. 6-Propyl-9-amino-5,6,6a,7-tetrahydro-4H-benzo[de]thiazolo [4,5-g]chinolin, seine Enantiomeren und die Säureadditionssalze

9. 6-Methyl-9-benzylamino-5,6,6a,7-tetrahydro-4H-benzo[de] thiazolo[4,5-g]chinolin,seine Enantiomeren und die Säureadditionssalze.

10. 6-Methyl-9-benzoyl-amino-5,6,6a,7-tetrahydro-4H-benzo[de] thiazolo [4,5-g]chinolin, seine Enantiomeren und die Säureadditionssalze

11. 6-Ethyl-9-methyl-amino-5,6,6a,7-tetrahydro-4H-benzo[de]thiazolo[4,5-g]chinolin, seine Enantiomeren und die Säureadditionssalze

12. 6-Ethyl-9-dimethylamino-5,6,6a,7-tetrahydro-4H-benzo[de] thiazolo[4,5-g]chinolin seine Enantiomere und die Säureadditionssalze

13. Arzneimittel, enthaltend als Wirkstoff eine Verbindung gemäß den Ansprüchen 1 bis 12 oder ein physiologisch verträgliches Säureadditionssalz neben Hilfs-und/oder Trägerstoffen und/oder Verdünnungsmitteln

14. Verwendung einer Verbindung gemäß den Ansprüchen 1 bis 12 oder eines physiologisch verträglichen Säureadditionssalzes zur Herstellung von Arzneimitteln bei der Behandlung von Erkrankungen, die auf präsynaptische Dopaminagonisten ansprechen.

15. Verwendung einer Verbindung oder eines physiologisch verträglichen Säureadditionssalzes gemäß Anspruch 14 zur Herstellung von Arzneimitteln bei der Behandlung der Schizophrenie.

16. Verwendung einer Verbindung gemäß den Ansprüchen 1 bis 12 oder eines physiologisch verträglichen Säureadditionssalzes zur Herstellung von Arzneimitteln bei der Behandlung der Parkinsonschenkrankeit und gegen Prolactin-Überproduktion.

17. Verfahren zur Herstellung der Verbindungen gemäß den Ansprüchen 1 bis 12 nach an sich bekannten Methoden, dadurch gekennzeichnet, daß

a. ein Keton der allgemeinen Formel

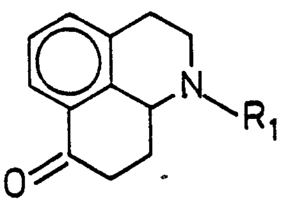

(III),

in der $R_1$ die Bedeutung gemäß Ansprüchen 1 bis 4 hat, bromiert wird und anschließend mit einem Thioharnstoff der Formel

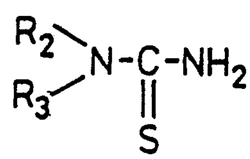

(IV),

in der

$R_2$ und $R_3$ die Bedeutung gemäß den Ansprüchen 1 bis 4 haben, umgesetzt wird,

oder daß man

b. eine Verbindung der Formel

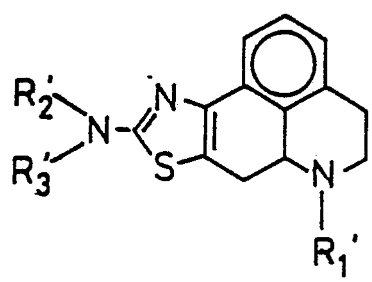

.(I'),

In der $R_1'$ die Bedeutung gemäß Ansprüchen 1 bis 4 hat und $R_2'$ und $R_3'$ Wasserstoff sind, mit einem Säurechlorid der Formel

$$R_4 - \underset{\underset{O}{\|}}{C} - Cl \quad ,$$

worin $R_4$ eine Alkyl-oder Phenylgruppe der für $R_2$ gemäß Ansprüchen 1 bis 4 geltenden Acyl-oder Phenylacylreste bedeutet, umsetzt

oder daß man

c. eine Verbindung der Formel

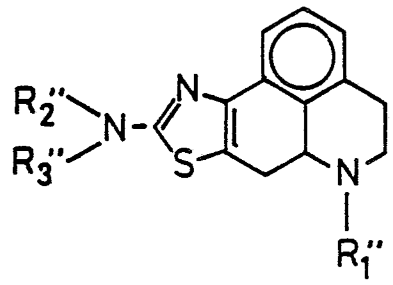

(I"),

worin $R_1$" die Bedeutung gemäß Ansprüche 1 bis 4 hat, $R_2$" die Acyl-oder Phenylacylreste und $R_3$" Wasserstoff sind, mit einem Metallhydrid zu einer entsprechenden N-Alkylamino-Verbindung reduziert

oder daß man

d. eine Verbindung der Formel

(I'''),

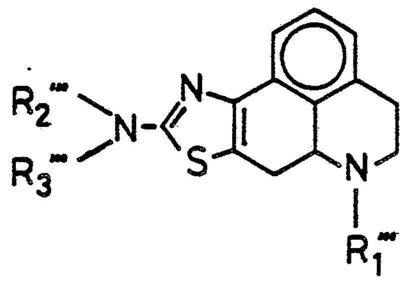

worin $R_1$''' ein Methyl-oder Benzylrest bedeutet und $R_2$''' und $R_3$''' die Bedeutung gemäß Ansprüchen 1 bis 4 haben, entmethyliert oder entbenzyliert

und daß gewünschtenfalls so erhaltene racemische Verbindungen nach an sich bekannten Methoden in ihre Enantiomeren aufgetrennt werden und/oder zunächst erhaltene Salze in freie Basen, zunächst erhaltene Basen in Säureadditionssalze übergeführt werden.